# FASCICULE DE BREVET EUROPEEN

(11) **EP 3 419 984 B1**
(45) Date de publication et mention de la délivrance du brevet: **15.12.2021**
(21) Numéro de dépôt: 17710358.7
(22) Date de dépôt: 24.02.2017
(51) Int. Cl.: C07H 1/08, C07H 3/02, C07H 3/06, C08B 37/00, C08H 8/00, C08L 5/00, C08L 5/14, C13K 1/02

(54) **PROCÉDÉ DE PRÉPARATION D'UN MÉLANGE DE MONOSACCHARIDES ET/OU D'OLIGOSACCHARIDES ET/OU DE POLYSACCHARIDES PAR PURIFICATION D'UN HYDROLYSAT DE MATIÈRES LIGNOCELLULOSIQUES**
VERFAHREN ZUR HERSTELLUNG EINER MISCHUNG AUS MONOSACCHARIDEN UND/ODER OLIGOSACCHARIDEN UND/ODER POLYSACCHARIDEN DURCH REINIGUNG EINES LIGNOCELLULOSEMATERIALIENHYDROLYSATS
PROCESS FOR THE PREPARATION OF A MIXTURE OF MONOSACCHARIDES AND/OR OLIGOSACCHARIDES AND/OR POLYSACCHARIDES BY PURIFICATION OF A HYDROLYSATE OF LIGNOCELLULOSIC MATERIALS

(30) Priorité: 25.02.2016 FR 1651569
(43) Date de publication de la demande: 02.01.2019
(73) Titulaire: Institut Polytechnique de Grenoble, 38031 Grenoble Cedex 1 (FR)
(72) Inventeur: CHIRAT, Christine, 38100 Grenoble (FR); LACHENAL, Dominique, 38130 Echirolles (FR); BOUCHER, Jérémy, 31800 Saint Gaudens (FR)
(74) Mandataire: Bronchart, Quentin
(86) Numéro de dépôt international: PCT/FR2017/050415
(87) Numéro de publication internationale: WO 2017/144829

(56) Documents cités:
- WO-A1-02/14598
- WO-A1-2006/032282
- WO-A2-2010/130889
- US-A1- 2012 021 467
- US-A1- 2015 184 260
- Anuj K Chandel ET AL: "Detoxification of Lignocellulosic Hydrolysates for Improved Bioethanol Production", Biofuel Production - Recent Developments and Prospects, 1 septembre 2011 (2011-09-01), pages 225-246, XP055209568, DOI: 10.5772/16454 ISBN: 978-9-53-307478-8 Extrait de l'Internet: URL:http://cdn.intechopen.com/pdfs-wm/2006 3.pdf [extrait le 2015-08-26]

## Description

### Domaine de l'invention

Le domaine de cette invention est celui de la valorisation des sucres issus des sous-produits des filières papier et cellulose et des bioraffineries lignocellulosiques.

En particulier, la présente invention concerne la préparation d'un mélange de monosaccharides et/ou d'oligosaccharides et/ou de polysaccharides par purification d'un hydrolysat de matières lignocellulosiques, ce dernier comprenant des hémicelluloses sous forme de monomères, d'oligomères et éventuellement de polymères.

### Arrière-plan technologique

La matière lignocellulosique est le constituant principal de la paroi cellulaire des plantes. Elle est composée de cellulose, d'hémicelluloses, de lignine et d'extractibles dans des proportions variables selon l'origine de la matière. Le bois représente la part majeure de la matière lignocellulosique disponible.

L'industrie papetière utilise le bois pour en extraire les fibres de cellulose et ainsi produire la pâte à papier. Selon un procédé standard, la cellulose est extraite du bois par un traitement alcalin à haute température (procédé Kraft). Les produits de dégradation des hémicelluloses, de la lignine et les autres sous-produits obtenus par ce traitement sont recueillis dans un effluent appelé liqueur noire. Cet effluent est pour l'instant valorisé uniquement énergétiquement, sa complexité ne permettant pas d'envisager une extraction des hémicelluloses, ces dernières étant majoritairement dégradées sous forme d'acides organiques.

La valorisation énergétique de la liqueur noire obtenue est intéressante car elle permet aux industries papetières de produire de l'énergie, et d'être autonomes, voire excédentaire, en énergie. Cependant, dans le contexte environnemental actuel, une autre valorisation des composés, autres que la cellulose, contenus dans le bois est souhaitable.

Les hémicelluloses du bois sont des polymères de petite taille (degré de polymérisation compris entre 50 et 200) composés de différents types de sucres, leur composition variant en fonction de la provenance. Parmi les sucres présents dans les hémicelluloses, on peut citer le mannose, le galactose, le glucose, le xylose et l'arabinose. De par leur composition, ces hémicelluloses permettent ainsi d'avoir accès à d'autres sucres que le glucose. Ces sucres sont particulièrement intéressants car il s'agit de sucres issus de matières premières non-alimentaires, par opposition au glucose obtenu à partir d'amidon (maïs, blé, pomme de terre).

Divers procédés ont donc été mis au point afin d'extraire au moins une partie des hémicelluloses du bois avant de réaliser l'extraction des fibres de cellulose par le procédé Kraft. Les hémicelluloses sont hydrolysées et obtenues principalement sous forme de monomères et d'oligomères. Jusqu'à maintenant les industriels qui extrayaient les hémicelluloses de copeaux de bois, n'étaient pas intéressés par la valorisation des hémicelluloses, mais uniquement par la production de la cellulose pure : pour ce faire ils soumettent les copeaux de bois à une auto hydrolyse consistant à injecter de la vapeur et traiter les copeaux à 170-180°C pendant 1 à 2 heures, ce qui permet de solubiliser une grande partie des hémicelluloses. Puis les copeaux et les hémicelluloses solubilisées sont soumis à la cuisson Kraft, ce qui permet de produire des fibres de cellulose, les hémicelluloses préalablement solubilisées se retrouvent dans la liqueur noire, et sont brûlées avec la lignine (Herbert Sixta, Handbook of Pulp, Wiley-VCH., 2006, 4.2.7.1. Prehydrolysis, p. 325).

Or étant donné le développement de la chimie biosourcée, la demande en sucres est de plus en plus forte, et il serait judicieux de récupérer les sucres issus des hémicelluloses du bois afin de les valoriser, ce qui représenterait pour les usines productrices de cellulose un revenu supplémentaire (Ragauskas AJ, Nagy M, Kim DH, Eckert CA, Hallett JP, Liotta CL, From wood to fuels-integrating biofuels and pulp production Industrial Biotechnology 2006; 2(1), 55-65).

Le document US2015/0184260 A1 décrit un procédé de production de sucres fermentescibles à partir d'un hydrolysat de bois comprenant des oligomères d'hémicelluloses et à partir d'oligomères de glucose extrait de la cellulose grâce à des enzymes. Les sucres obtenus peuvent ainsi être utilisés pour la production de biocarburant ou de produits biochimiques.

Le document EP 2067793 A1 décrit l'utilisation d'un hydrolysat de bois, obtenu à partir d'un traitement hydrothermal de copeaux de bois, pour la production de polymères biodégradables et renouvelables ainsi que de différents produits tels que des films ou des gels.

Le document WO 02/14598 A1 décrit un procédé de traitement de biomasse lignocellulosique, séparant les fibres de cellulose des autres constituants de la biomasse, afin de produire de la cellulose purifiée contenant moins de 20 % de lignine.

Le document WO 2006/032282 A1 décrit un procédé de traitement d'un matériau, constitué de biomasse ou de déchets organiques, contenant un matériau lignocellulosique, visant à améliorer la production, par hydrolyse enzymatique et/ou fermentation, d'éthanol, d'hydrogène, d'acide lactique, de méthane, de succinate ou d'acides organiques.

Cependant, il reste difficile d'extraire et de purifier les sucres contenus dans les hydrolysats de bois obtenus par traitement d'hydrolyse. En effet, ces hydrolysats contiennent, outre les sucres des hémicelluloses sous forme de monomères et d'oligomères, des produits de dégradation des sucres tels que le furfural et l'hydroxyméthylfurfural, de la lignine précipitée et dissoute, certains extractibles du bois, des composés polymérisés (qui peuvent provenir de réactions entre la lignine et des furanes, voire entre la lignine et des extractibles, ou de la polymérisation des furanes entre eux) et des acides organiques.

La présence de ces différents composés pose des problèmes à plusieurs niveaux pour la suite du traitement des hydrolysats. La présence de lignine et/ou d'autres composés polymérisés (autres que les polysaccharides ou oligosaccharides) induit des dépôts polymériques collants (DPC) sur les équipements utilisés, ce qui requiert un niveau de maintenance des équipements trop élevé et rend difficile l'application d'un procédé de traitement des hydrolysats à l'échelle industrielle. Il y a également des problèmes de purification des sucres obtenus à cause de la présence du furfural et d'hydroxyméthylfurfural dans l'hydrolysat.

Ces problèmes représentent un réel frein à la valorisation chimique des sucres issus des hémicelluloses et à l'industrialisation des procédés d'extraction et de traitement de ces sucres.

### Objectifs

Dans ce contexte, la présente invention vise à satisfaire au moins l'un des objectifs suivants.

L'un des objectifs essentiels de l'invention est de fournir un nouveau procédé de purification d'un hydrolysat de matières lignocellulosiques.

Un autre objectif essentiel de l'invention est de fournir un nouveau procédé de purification d'un hydrolysat de matières lignocellulosiques sans pour autant diminuer les quantités de sucres présents dans cet hydrolysat.

Un autre objectif essentiel de l'invention est de fournir un nouveau procédé qui permette de réduire la quantité de furfural et/ou d'hydroxyméthylfurfural contenu dans un hydrolysat de matières lignocellulosiques.

Un autre objectif essentiel de l'invention est de fournir un nouveau procédé qui permette de réduire la masse moléculaire en poids et/ou en nombre des polymères, autres que les hémicelluloses, contenus dans un hydrolysat de matières lignocellulosiques.

Un autre objectif essentiel de l'invention est de fournir un mélange de monosaccharides et/ou d'oligosaccharides et/ou de polysaccharides qui puisse être purifié facilement.

Un autre objectif essentiel de l'invention est de fournir un mélange de monosaccharides et/ou d'oligosaccharides et/ou de polysaccharides ayant une quantité de furfural et/ou d'hydroxyméthylfurfural réduite.

Un autre objectif essentiel de l'invention est de fournir un mélange de monosaccharides et/ou d'oligosaccharides et/ou de polysaccharides comprenant des polymères ayant une masse moléculaire en poids et/ou en nombre réduite.

Un autre objectif essentiel de l'invention est de fournir un mélange de monosaccharides et/ou d'oligosaccharides et/ou de polysaccharides qui pose moins, voire aucun, problème de dépôts sur les équipements.

Un autre objectif essentiel de l'invention est de fournir un mélange de monosaccharides et/ou d'oligosaccharides et/ou de polysaccharides qui pose moins, voire aucun, problème de purification des sucres.

### Brève description de l'invention

Ces objectifs, parmi d'autres, sont atteints par la présente invention qui concerne en premier lieu un procédé de préparation d'un mélange de monosaccharides et/ou d'oligosaccharides et/ou de polysaccharides par purification d'un hydrolysat de matières lignocellulosiques, ledit hydrolysat comprenant des hémicelluloses sous forme de monomères, d'oligomères et éventuellement de polymères, le procédé comprenant au moins une étape d'oxydation dudit hydrolysat avec au moins un oxydant, et étant caractérisé en ce que l'étape d'oxydation est effectuée à une température comprise entre 20 et 100°C.

De façon surprenante, il a été découvert que traiter un hydrolysat de matières lignocellulosiques avec un oxydant à une température comprise entre 20 et 100°C permettait de réduire la masse moléculaire en poids et en nombre des polymères précipités ou dissouts et de réduire la quantité de furfural et d'hydroxyméthylfurfural (HMF) contenus dans l'hydrolysat, sans pour autant diminuer ou détériorer les quantités de sucres dans l'hydrolysat de manière significative, comme on aurait pu le craindre.

Ce procédé permet d'obtenir un mélange de monosaccharides et/ou d'oligosaccharides et/ou de polysaccharides qui peut être utilisé industriellement et dont les sucres peuvent être séparés et purifiés facilement. On arrive ainsi à valoriser les sucres issus de l'hémicellulose sans compromettre le procédé papetier de production de fibres de cellulose.

L'invention concerne également un procédé de préparation d'un mélange de monosaccharides et/ou d'oligosaccharides et/ou de polysaccharides, par purification d'un hydrolysat de matières lignocellulosiques issu d'un traitement thermique de matières lignocellulosiques en présence d'eau ou de vapeur d'eau suivi d'une séparation du co-produit constitué par un résidu riche en cellulose et éventuellement en lignine, ledit hydrolysat comprenant des hémicelluloses sous forme de monomères et d'oligomères, et éventuellement de polymères, caractérisé en ce que le procédé comprend au moins une étape d'oxydation dudit hydrolysat avec au moins un oxydant.

L'invention concerne dans un second lieu un mélange de monosaccharides et/ou d'oligosaccharides et/ou de polysaccharides obtenu par purification d'un hydrolysat de matières lignocellulosiques.

### Définitions :

Dans le présent exposé, tout singulier désigne indifféremment un singulier et un pluriel et réciproquement, sauf indication contraire expresse. Les définitions suivantes sont données à titre d'exemples pour l'interprétation du présent exposé.

*Matières lignocellulosiques* : elles sont le constituant principal de la paroi cellulaire des plantes et sont composés principalement de cellulose, d'hémicelluloses, de lignine et d'extractibles. Ces matières peuvent provenir, entre autres, du bois, du bois recyclé, des plantes annuelles, des résidus agricoles (bagasse, pailles, ...), ou des papiers et cartons récupérés.

*Hydrolysat de matières lignocellulosiques* : phase liquide obtenue par traitement thermique de matières lignocellulosiques en présence d'eau ou de vapeur d'eau. Ce traitement peut être effectué uniquement avec de l'eau ou de la vapeur d'eau, mais aussi en milieu acide par ajout d'un acide faible ou fort, ou en milieu légèrement alcalin (Maki-Arvela P, Salmi T, Holmbom B, Willfor S and Yu D. Murzin, Synthesis of Sugars by Hydrolysis of Hemicelluloses- A Review, Chem. Rev. 2011, 111, 5638-5666, dx.doi.org/10.1021/cr2000042). Il permet la séparation d'un hydrolysat et d'un résidu riche en cellulose et éventuellement en lignine. Ce traitement conduit, d'une part, à l' *"Hydrolysat de matières lignocellulosiques",* et, d'autre part, à un résidu riche en cellulose et éventuellement en lignine. Ce résidu peut ensuite être séparé de l'hydrolysat et être utilisé pour fabriquer de la pâte à papier ou de la cellulose pour différents usages (textile, chimique, ou des nanocristaux ou microfibrilles de cellulose par exemple).

*Oligomères :* au sens de l'invention, on entend par « oligomères » des molécules comprenant un nombre de motifs monomères compris entre 2 et 25.

*Polymères* : au sens de l'invention, on entend par « polymères » des molécules comprenant un nombre de motifs monomères supérieur à 25.

*Composés colorés* : au sens de l'invention, on entend par « composés colorés » des composés qui présentent un signal d'absorbance en spectroscopie d'absorption dans l'UV-visible entre 200 et 400 nm. Il s'agit, entre autres, des polymères issus des produits de dégradation des composants du bois.

### Brève description des dessins

La figure 1 représente l'effet des traitements au peroxyde d'hydrogène sur les teneurs en furfural et hydroxyméthylfurfural de l'hydrolysat.
La figure 2 représente l'effet des traitements au peroxyde d'hydrogène sur les teneurs en sucres en C5 et en C6 totaux (monomères + oligomères) de l'hydrolysat.
La figure 3 représente l'effet du traitement à l'ozone sur les teneurs en furfural et en hydroxyméthylfurfural de l'hydrolysat.
La figure 4 représente l'effet du traitement à l'ozone sur les teneurs en sucres en C5 et en C6 totaux (monomères + oligomères) de l'hydrolysat.

### Description détaillée de l'invention

### Hydrolysat de matières lignocellulosiques :

L'hydrolysat est obtenu par traitement thermique de toutes sortes de matières lignocellulosiques en présence d'eau ou de vapeur d'eau. Ce traitement en présence d'eau ou de vapeur d'eau permet la préparation de l'hydrolysat et d'un résidu riche en cellulose, et éventuellement en lignine. Avantageusement, ce résidu est ensuite séparé de l'hydrolysat, par exemple par filtration. Selon un mode de réalisation, les matières lignocellulosiques sont choisies parmi le bois, le bois recyclé, les plantes annuelles, les résidus agricoles, les papiers et cartons récupérés et la bagasse, de préférence le bois.

Selon un mode de réalisation de l'invention, l'hydrolysat de matières lignocellulosiques est obtenu par hydrolyse en utilisant de l'eau ou de la vapeur additionnée d'un acide faible ou fort.

L'hydrolysat de matières lignocellulosiques comprend des hémicelluloses sous forme de monomères, d'oligomères ou de polymères. Il comprend également des produits de dégradation des sucres, tels que l'hydroxyméthylfurfural et le furfural, et des produits issus de la lignine et/ou issus des produits de dégradation des composants du bois (ces produits peuvent être soit sous forme dissoute, soit sous forme précipitée). Il peut également contenir des acides organiques, ainsi que des produits issus des extractibles du bois.

Selon un mode de réalisation de l'invention, l'hydrolysat de matières lignocellulosiques a une concentration totale en sucres comprises entre 15 et 45 g/L.

### Oxydant

Le procédé comprend au moins une étape d'oxydation avec au moins un oxydant. Selon un mode de réalisation, la quantité d'oxydant utilisée est comprise entre 1 et 100% en masse par rapport à la quantité de lignine (précipitée et dissoute), de furfural, d'hydroxyméthylfurfural et des composés colorés présents dans l'hydrolysat.

Selon un mode particulier de réalisation de l'invention, l'oxydant est choisi parmi le peroxyde d'hydrogène, l'ozone, l'oxygène, les peracides, les radicaux libres et les oxydants chlorés tel que le dioxyde de chlore.

De préférence, l'oxydant est choisi parmi le peroxyde d'hydrogène et l'ozone.

Selon un mode de réalisation de l'invention, l'oxydation est effectuée avec une combinaison de deux oxydants, par exemple du peroxyde d'hydrogène et de l'ozone ou du peroxyde d'hydrogène et de l'oxygène.

### Méthodologie

Selon un mode de réalisation préféré de l'invention, le procédé suivant l'invention comprend les étapes suivantes :
- mise en œuvre d'un hydrolysat de matières lignocellulosiques ;
- éventuellement, ajustement du pH, pour obtenir un pH compris entre 3,5 et 12 ;
- mise en contact de l'hydrolysat avec au moins un oxydant ;
- chauffage du milieu réactionnel à une température comprise entre 20 et 100°C, de préférence à une température inférieure ou égale à [en °C et dans un ordre croissant de préférence] : 100 ; 90 ; 80 ; 70 ;
- récupération d'un mélange de monosaccharides et/ou d'oligosaccharides et/ou de polysaccharides contenant moins de furfural et d'hydroxyméthylfurfural et dont la masse moléculaire des polymères de lignine et/ou de produits de dégradation a été réduite ;
- éventuellement, neutralisation de l'oxydant résiduel.

Selon un mode de réalisation, le procédé suivant l'invention comprend les étapes suivantes :
- traitement thermique de matières lignocellulosiques en présence d'eau ou de vapeur d'eau pour obtenir un hydrolysat de matières lignocellulosiques et un résidu riche en cellulose, et éventuellement en lignine;
- séparation de l'hydrolysat de matières lignocellulosiques du résidu riche en cellulose, par exemple par filtration ;
- éventuellement, ajustement du pH de l'hydrolysat, pour obtenir un pH compris entre 3,5 et 12 ;
- mise en contact de l'hydrolysat avec au moins un oxydant ;
- chauffage du milieu réactionnel à une température comprise entre 20 et 100°C, de préférence à une température inférieure ou égale à [en °C et dans un ordre croissant de préférence] 100 ; 90 ; 80 ; 70
- récupération d'un mélange de monosaccharides et/ou d'oligosaccharides et/ou de polysaccharides contenant moins de furfural et d'hydroxyméthylfurfural et dont la masse moléculaire des polymères de lignine et/ou de produits de dégradation a été réduite ;
- éventuellement, neutralisation de l'oxydant résiduel.

Avantageusement, l'étape d'oxydation est effectuée à un pH compris entre 1 et 13, de préférence entre 3,5 et 11.

Selon un mode de réalisation de l'invention, lorsque l'oxydant est le peroxyde d'hydrogène, la réaction est effectuée à un pH compris entre 8 et 12.

Avantageusement, le pH est ajusté par ajout de soude au milieu réactionnel.

Selon un autre mode de réalisation de l'invention, lorsque l'oxydant est l'ozone, la réaction est effectuée à un pH compris entre 2 et 5.

Selon un mode particulier de réalisation de l'invention, le pH n'est pas modifié et l'étape d'oxydation est effectuée au pH de l'hydrolysat de matières lignocellulosiques, c'est-à-dire entre 3 et 3,5.

De préférence, l'étape d'oxydation est effectuée à une température inférieure ou égale à [en °C et dans un ordre croissant de préférence] : 100 ; 90 ; 80 ; 70 ; comprise entre 20 et 100°C. La température à laquelle l'étape d'oxydation est effectuée est un paramètre important parmi d'autres. En effet, il est préférable que cette température ne soit pas trop élevée, afin d'éviter une dégradation des sucres monomères et oligomères contenus dans l'hydrolysat.

Selon un mode de réalisation de l'invention, lorsque l'oxydant est l'ozone, la réaction est effectuée à température ambiante.

Selon un mode particulier de réalisation de l'invention, le traitement oxydant dure entre quelques minutes et 4 heures, de préférence entre quelques minutes et 2 heures.

La réaction d'oxydation est par exemple effectuée à pression atmosphérique. La réaction d'oxydation peut également être effectuée sous pression. Par exemple, la réaction peut être effectuée à une pression inférieure à 10 bars ou comprise entre 5 et 10 bars.

### Mélange de monosaccharides et/ou d'oligosaccharides et/ou de polysaccharides

Ce procédé permet d'obtenir un mélange de monosaccharides et/ou d'oligosaccharides et/ou de polysaccharides comprenant éventuellement des traces résiduelles d'oxydant et des acides organiques.

Ce procédé permet d'obtenir un mélange de monosaccharides et/ou d'oligosaccharides et/ou de polysaccharides comprenant des polymères issus de la lignine, des extractibles du bois et/ou des produits de dégradation des hémicelluloses, ayant une masse moléculaire en poids et/ou en nombre réduite d'au moins 10%, de préférence 25% et encore plus préférentiellement 50%, par rapport à ces mêmes polymères contenus dans l'hydrolysat de matières lignocellulosiques.

Ce procédé permet d'obtenir un mélange de monosaccharides et/ou d'oligosaccharides et/ou de polysaccharides comprenant une quantité de furfural et d'hydroxyméthylfurfural réduite d'au moins 10%, de préférence 25% et encore plus préférentiellement 50%, par rapport à la quantité de furfural et d'hydroxymethylfurfural présente dans l'hydrolysat de matières lignocellulosiques.

### Applications :

Le mélange de monosaccharides et/ou d'oligosaccharides et/ou de polysaccharides obtenu après traitement peut être utilisé industriellement et les sucres contenus dans ce mélange peuvent être séparés et purifiés.

### EXEMPLES :

L'hydrolysat de matières lignocellulosiques et le mélange de monosaccharides et/ou d'oligosaccharides et/ou de polysaccharides sont analysés par chromatographie haute performance d'échange d'anions à haute pression couplée à un détecteur à ampérométrie pulsée (HPAEC-PAD, pour High Performance Anion Exchange Chromatography - Pulsed Amperometric Detection). Le système de chromatographie est un modèle Dionex ICS 5000, composé d'une colonne de garde (50 mm × 4 mm), d'une colonne CarboPac PA10 (250 mm × 4 mm) et d'un détecteur à ampérométrie pulsée composé d'une électrode de référence et d'une électrode d'or.

### 1) Préparation de l'hydrolysat de matières lignocellulosiques.

L'hydrolysat de matières lignocellulosiques a été obtenu par autohydrolyse de 400 g (exprimé en matière sèche) de copeaux de bois de feuillus dans les conditions suivantes :
- Ratio Liquide/Bois sec = 2
- Température : 170°C
- Temps de montée en température : 30 min
- Temps à température : 120 min
Temps de descente en température (jusqu'à 50°C) : 30 min

L'hydrolysat a ensuite été séparé des copeaux par filtration.

La composition en sucres de l'autohydrolysat obtenu est présentée dans le tableau 1. Son pH est de 3,5.

**Tableau 1 : composition en sucres de l'hydrolysat de matières lignocellulosiques**

| | Quantité (g/L) | | |
|---|---|---|---|
| Sucre | Monomères | Oligomères | Total |
| **Arabinose** | 0,6 | 0,0 | 0,6 |
| **Galactose** | 1,7 | 0,4 | 2,1 |
| **Glucose** | 1,2 | 1,6 | 2,8 |
| **Xylose** | 12,9 | 5,9 | 18,8 |
| **Mannose** | 1,3 | 2,1 | 3,4 |
| Total Pentoses | **13,5** | **5,9** | **19,1** |
| Total Hexoses | **4,2** | **4,1** | **8,3** |
| Total sucres | **17,7** | **10,0** | **27,7** |

Les autres composants de l'hydrolysat de matières lignocellulosiques obtenu sont les suivants :
- la quantité d'hydroxyméthylfurfural (HMF) est de 0,6 g/L,
- la quantité de furfural est de 6,3 g/L.
- la quantité de lignine et composés colorés solubles est de 8,6 g/L,
- la quantité de lignine et composés colorés insolubles est de 5,6 g/L.

La teneur totale en autres composants est donc de 21,1 g/L.

### 2) Traitement de l'hydrolysat avec du peroxyde d'hydrogène

Un volume de 50 ml de l'hydrolysat obtenu précédemment est utilisé tel quel. Le pH de l'hydrolysat est d'abord ajusté si besoin avec de la soude puis l'hydrolysat est mis en contact avec une solution commerciale diluée de peroxyde d'hydrogène. La concentration en peroxyde d'hydrogène dans l'hydrolysat est fixée à 16g/L. Puis la solution est chauffée à la température désirée pendant un temps donné. Les conditions sont résumées dans le tableau 2.

**Tableau 2 : Conditions des différents essais effectués avec du peroxyde d'hydrogène**

| N° essai | pH initial | Température (°C) | Durée (h) |
|---|---|---|---|
| 1 | 3,5 | 70 | 2 |
| 2 | 10 | 70 | 2 |
| 3 | 3,5 | 80 | 4 |
| 4 | 12 | 80 | 4 |
| 5 | 12 | 90 | 4 |

Le mélange de monosaccharides et/ou d'oligosaccharides et/ou de polysaccharides obtenu après traitement est analysé.

Le tableau 3 résume les effets du traitement oxydants sur les moyennes des masses moléculaires en nombre (Mn) et en poids (Mw) de la lignine et des composés colorés insolubles et solubles. Selon les conditions de traitement on observe une baisse de la masse moléculaire en nombre pouvant aller jusqu'à 79%, et de la masse moléculaire en poids jusqu'à 76%.

**Tableau 3. Evolution des masses moléculaires en nombre (Mn) et en poids (Mw) de la lignine et des matières colorées non solubles et solubles en fonction des différents traitements.**

| Traitement (référence essai, cf tableau 2) | Effet sur la lignine et composés colorés insolubles | | Effet sur la lignine et composés colorés solubles | |
|---|---|---|---|---|
| | ΔMn/Mn,% | ΔMw/Mw, % | ΔMn/Mn,% | ΔMw/Mw, % |
| 1 | 0 | 27 | 32 | 13 |
| 2 | 0 | 29 | 42 | 50 |
| 3 | 0 | 38 | 52 | 18 |
| 4 | 27 | 63 | 77 | 73 |
| 5 | 27 | 52 | 79 | 76 |

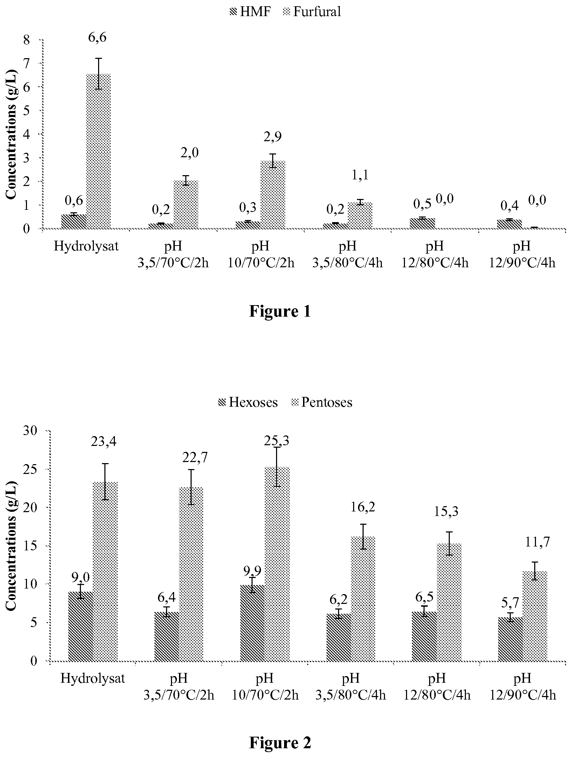

L'effet des traitements au peroxyde d'hydrogène sur le furfural et le HMF a été mesuré (Figure 1). La teneur en furfural et en HMF est abaissée de plus de 50% dans les conditions opératoires les plus douces (pH 3,5, 70°C, 2h et pH 10, 70°C, 2hr). Dans les conditions les plus fortes, la concentration en furfural est pratiquement nulle. Les concentrations en HMF, déjà faibles au départ, peuvent être divisées par 3.

La composition en sucres a été vérifiée après traitement. Les résultats présentés sur la figure 2 montrent que dans les deux conditions les plus douces (pH 3,5, 70°C, 2 hrs, et pH 10, 70°C, 2 hr), aucun impact significatif n'est décelé sur les pentoses, ni sur les hexoses pour l'essai en milieu alcalin.

### 3) Traitement de l'hydrolysat à l'ozone

Les traitements à l'ozone ont été effectués au pH de l'hydrolysat, soit environ 3-3,5, à température ambiante, avec des charges d'ozone croissantes entre 4 et 16 g d'ozone introduit / litre d'hydrolysat.

Les mêmes analyses que pour le traitement au peroxyde d'hydrogène ont été effectuées. Le tableau 4 montre que le traitement à l'ozone est efficace dès les faibles charges en ozone ajoutées.

**Tableau 4. Evolution des masses moléculaires en nombre (Mn) et en poids (Mw) de la lignine et des matières colorées non solubles et solubles en fonction des différents traitements à l'ozone.**

| Traitement à l'ozone | Effet sur la lignine et composés colorés insolubles | | Effet sur la lignine et composés colorés solubles | |
|---|---|---|---|---|
| Charge en ozone, g/L | ΔMn/Mn,% | ΔMw/Mw, % | ΔMn/Mn,% | ΔMw/Mw, % |
| 4 | 0 | 50 | 62 | 46 |
| 8 | 0 | 35 | 64 | 44 |
| 12 | 0 | 30 | 59 | 43 |
| 16 | 0 | 67 | 60 | 40 |

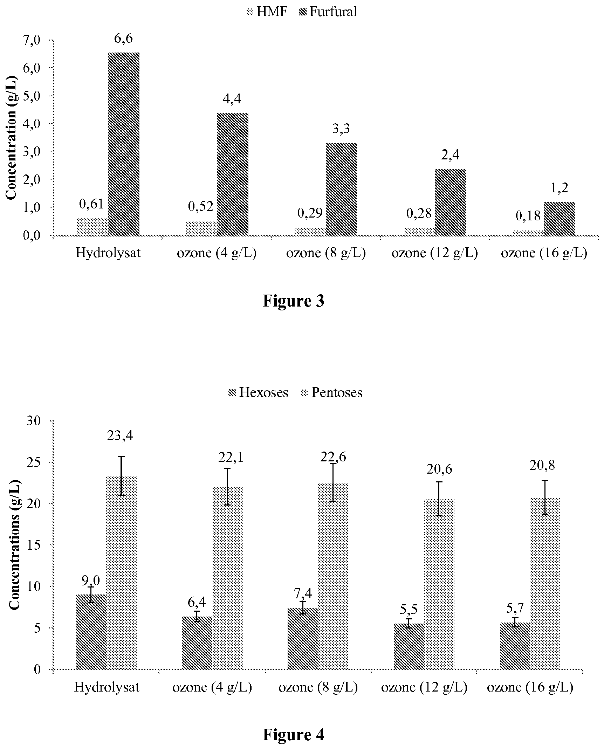

L'ozone conduit également à la réduction des quantités en furfural et HMF de manière significative (Figure 3).

En ce qui concerne l'impact sur les concentrations en sucres (Figure 4), il est intéressant de constater que l'ozone n'a pas d'effet sur les pentoses jusqu'à 8g/L, puis les concentrations ne baissent que très légèrement, ce qui est surprenant étant donné que l'ozone est connu pour oxyder les sucres et les dégrader.

## Revendications

1. Procédé de préparation d'un mélange de monosaccharidcs et/ou d'oligosaccharides et/ou de polysaccharides, par purification d'un hydrolysat de matières lignocellulosiques, ledit hydrolysat comprenant des hémicelluloses sous forme de monomères et d'oligomères, et éventuellement de polymères, le procédé comprenant au moins une étape d'oxydation dudit hydrolysat avec au moins un oxydant, et étant **caractérisé en ce que** l'étape d'oxydation est effectuée à une température comprise entre 20 et 100°C.

2. Procédé selon la revendication 1 **caractérisé en ce que** les matières lignocellulosiques sont choisies parmi le bois, le bois recyclé, les plantes annuelles, les résidus agricoles et les papiers et cartons récupérés, de préférence le bois.

3. Procédé selon la revendication 1 ou 2 **caractérisé en ce que** ledit hydrolysat comprend également des produits de dégradation des sucres, des extractibles du bois et des polymères issus de la lignine et/ou issus des produits de dégradation des sucres.

4. Procédé selon l'une quelconque des revendications précédentes **caractérisé en ce que** la quantité d'oxydant utilisée est comprise entre 1 et 100% en masse par rapport à la quantité de lignine (précipitée et dissoute), de furfural, d'hydroxyméthylfurfural et de composés colorés présents dans l'hydrolysat.

5. Procédé selon l'une quelconque des revendications précédentes **caractérisé en ce que** l'oxydant est choisi parmi le peroxyde d'hydrogène, l'ozone, les peracides, les radicaux libres et les oxydants chlorés.

6. Procédé selon l'une quelconque des revendications précédentes **caractérisé en ce que** l'oxydant est choisi parmi l'ozone et le peroxyde d'hydrogène.

7. Procédé selon l'une quelconque des revendications précédentes **caractérisé en ce que** l'étape d'oxydation est effectuée à un pH compris entre 1 et 13, de préférence entre 3,5 et 11.

8. Procédé selon l'une quelconque des revendications précédentes **caractérisé en ce qu'**il comprend les étapes suivantes :
- mise en œuvre d'un hydrolysat de matières lignocellulosiques ;
- éventuellement, ajustement du pH, pour obtenir un pH compris entre 3,5 et 12 ;
- mise en contact de l'hydrolysat avec au moins un oxydant ;
- chauffage du milieu réactionnel, à une température comprise entre 20 et 100°C, de préférence à une température inférieure ou égale à [en °C et dans un ordre croissant de préférence] : 100 ; 90 ; 80 ; 70 ;
- récupération d'un mélange de monosaccharides et/ou d'oligosaccharides et/ou de polysaccharides contenant moins de furfural et d'hydroxyméthylfurfural et dont la masse moléculaire des polymères de lignine et/ou de produits de dégradation a été réduite ;
- éventuellement, neutralisation de l'oxydant résiduel.

9. Procédé selon l'une quelconque des revendications 1 à 7 **caractérisé en ce qu'**il comprend les étapes suivantes :
- traitement thermique de matières lignocellulosiques en présence d'eau ou de vapeur d'eau pour obtenir un hydrolysat de matières lignocellulosiques et un résidu riche en cellulose, et éventuellement en lignine;
- séparation de l'hydrolysat de matières lignocellulosiques du résidu riche en cellulose, par exemple par filtration ;
- éventuellement, ajustement du pH de l'hydrolysat, pour obtenir un pH compris entre 3,5 et 12 ;
- mise en contact de l'hydrolysat avec au moins un oxydant ;
- chauffage du milieu réactionnel, à une température comprise entre 20 et 100°C, de préférence à une température inférieure ou égale à [en °C et dans un ordre croissant de préférence] : 100 ; 90 ; 80 ; 70 ;
- récupération d'un mélange de monosaccharides et/ou d'oligosaccharides et/ou de polysaccharides contenant moins de furfural et d'hydroxyméthylfurfural et dont la masse moléculaire des polymères de lignine et/ou de produits de dégradation a été réduite ;
- éventuellement, neutralisation de l'oxydant résiduel.

10. Procédé selon l'une quelconque des revendications précédentes **caractérisé en ce que** la réaction s'effectue au pH de l'hydrolysat de matières lignocellulosiques.

11. Mélange de monosaccharides et/ou d'oligosaccharides et/ou de polysaccharides susceptible d'être obtenu par purification d'un hydrolysat de matières lignocellulosiques selon l'une quelconque des revendications 1 à 10 **caractérisé en ce qu'**il contient des polymères issus de la lignine, des extractibles du bois et/ou des produits de dégradation des sucres, ayant une masse moléculaire en poids et/ou en nombre réduite d'au moins 10%, de préférence 25% et encore plus préférentiellement 50%, par rapport aux polymères contenus dans l'hydrolysat de matières lignocellulosiques.

12. Mélange de monosaccharides et/ou d'oligosaccharides et/ou de polysaccharides selon la revendication 11 **caractérisé en ce qu'**il comprend une quantité de furfural et d'hydroxyméthylfurfural réduite d'au moins 10%, de préférence 25% et encore plus préférentiellement 50%, par rapport à la quantité de furfural et d'hydroxyméthylfurfural présente dans l'hydrolysat de matières lignocellulosiques.

## Patentansprüche

1. Verfahren zur Herstellung einer Monosaccharid- und/oder Oligosaccharid- und/oder Polysaccharidmischung durch Reinigung eines Hydrolysats aus lignozellulosehaltigen Materialien, wobei das Hydrolysat Hemizellulosen in Form von Monomeren und von Oligomeren, und eventuell von Polymeren umfasst, wobei das Verfahren mindestens einen Oxidationsschritt des Hydrolysats mit mindestens einem Oxidationsmittel umfasst, und **dadurch gekennzeichnet ist, dass** der Oxidationsschritt bei einer Temperatur durchgeführt wird, die zwischen 20 und 100°C liegt.

2. Verfahren nach Anspruch 1, **dadurch gekennzeichnet, dass** die lignozellulosehaltigen Materialien aus Holz, recyceltem Holz, Einjahrespflanzen, landwirtschaftlichen Rückständen, und gesammeltem Papier und Karton, vorzugsweise aus Holz ausgewählt sind.

3. Verfahren nach Anspruch 1 oder 2, **dadurch gekennzeichnet, dass** das Hydrolysat auch Abbauprodukte von Zuckern, extrahierbare Stoffe aus dem Holz und aus Lignin stammende und/oder aus den Abbauprodukten der Zucker stammende Polymere umfasst.

4. Verfahren nach einem der vorstehenden Ansprüche, **dadurch gekennzeichnet, dass** die verwendete Menge an Oxidationsmittel zwischen 1 und 100 Ma.-% in Bezug auf die Menge an (abgeschiedenem und aufgelöstem) Lignin, an Furfural, an Hydroxymethylfurfural und an farbigen Verbindungen liegt, die in dem Hydrolysat vorhanden sind.

5. Verfahren nach einem der vorstehenden Ansprüche, **dadurch gekennzeichnet, dass** das Oxidationsmittel aus Wasserstoffperoxid, Ozon, Persäuren, freien Radikalen und chlorierten Oxidationsmitteln ausgewählt wird.

6. Verfahren nach einem der vorstehenden Ansprüche, **dadurch gekennzeichnet, dass** das Oxidationsmittel aus Ozon und Wasserstoffperoxid ausgewählt wird.

7. Verfahren nach einem der vorstehenden Ansprüche, **dadurch gekennzeichnet, dass** der Oxidationsschritt mit einem pH-Wert ausgeführt wird, der zwischen 1 und 13, vorzugsweise zwischen 3,5 und 11 liegt.

8. Verfahren nach einem der vorstehenden Ansprüche, **dadurch gekennzeichnet, dass** es die folgenden Schritte umfasst:
- Anwenden eines Hydrolysats aus lignozellulosehaltigen Materialien;
- eventuell Anpassen des pH-Werts, um einen pH-Wert zu erhalten, der zwischen 3,5 und 12 liegt;
- Kontaktieren des Hydrolysats mit mindestens einem Oxidationsmittel;
- Erhitzen des Reaktionsmediums auf eine Temperatur, die zwischen 20 und 100°C liegt, vorzugsweise auf eine Temperatur kleiner oder gleich [in °C und in aufsteigender Reihenfolge der Präferenz]: 100; 90; 80; 70;
- Auffangen einer Monosaccharid- und/oder Oligosaccharid- und/oder Polysaccharidmischung, die mindestens Furfural und Hydroxymethylfurfural enthält, und deren Molekulargewicht der Polymere aus Lignin und/oder Abbauprodukten reduziert worden ist;
- eventuell Neutralisieren des restlichen Oxidationsmittels.

9. Verfahren nach einem der Ansprüche 1 bis 7, **dadurch gekennzeichnet, dass** es die folgenden Schritte umfasst:
- Wärmebehandeln der lignozellulosehaltigen Materialien in Gegenwart von Wasser oder von Wasserdampf, um ein Hydrolysat aus lignozellulosehaltigen Materialien und einen zellulose- und eventuell ligninreichen Rest zu erhalten;
- Trennen des Hydrolysats aus lignozellulosehaltigen Materialien vom zellulosereichen Rest, beispielsweise durch Filtern;
- eventuell Anpassen des pH-Werts des Hydrolysats, um einen pH-Wert zu erhalten, der zwischen 3,5 und 12 liegt;
- Kontaktieren des Hydrolysats mit mindestens einem Oxidationsmittel;
- Erhitzen des Reaktionsmediums auf eine Temperatur, die zwischen 20 und 100°C liegt, vorzugsweise auf eine Temperatur kleiner oder gleich [in °C und in aufsteigender Reihenfolge der Präferenz]: 100; 90; 80; 70;
- Auffangen einer Monosaccharid- und/oder Oligosaccharid- und/oder Polysaccharidmischung, die weniger Furfural und Hydroxymethylfurfural enthält, und deren Molekulargewicht der Polymere aus Lignin und/oder Abbauprodukten reduziert worden ist;
- eventuell Neutralisieren des restlichen Oxidationsmittels.

10. Verfahren nach einem der vorstehenden Ansprüche, **dadurch gekennzeichnet, dass** die Reaktion mit dem pH-Wert des Hydrolysats aus lignozellulosehaltigen Materialien durchgeführt wird.

11. Monosaccharid- und/oder Oligosaccharid- und/oder Polysaccharidmischung, die durch Reinigung eines Hydrolysats aus lignozellulosehaltigen Materialien nach einem der Ansprüche 1 bis 10 erhalten werden kann, **dadurch gekennzeichnet, dass** es aus dem Lignin, aus den extrahierbaren Stoffen des Holzes und/oder aus den Abbauprodukten der Zucker stammende Polymere enthält, die ein Molekulargewicht in Gewicht und/oder in Anzahl aufweisen, das um mindestens 10 %, vorzugsweise 25 %, und noch bevorzugter 50 % in Bezug auf die Polymere reduziert ist, die in dem Hydrolysat aus lignozellulosehaltigen Materialien vorhanden sind.

12. Monosaccharid- und/oder Oligosaccharid- und/oder Polysaccharidmischung nach Anspruch 11, **dadurch gekennzeichnet, dass** sie eine Menge an Furfural und an Hydroxymethylfurfural umfasst, die um mindestens 10 %, vorzugsweise 25 %, und noch bevorzugter 50 % in Bezug auf die Menge an Furfural und an Hydroxymethylfurfural reduziert ist, die in dem Hydrolysat aus lignozellulosehaltigen Materialien vorhanden sind.

## Claims

1. A process for the preparation of a mixture of monosaccharides and/or oligosaccharides and/or polysaccharides, by purification of a hydrolysate of lignocellulosic materials, said hydrolysate comprising hemicelluloses in the form of monomers and oligomers, and optionally of polymers, the process comprising at least one step of oxidising said hydrolysate with at least one oxidant, and being **characterised in that** the oxidation step is performed at a temperature comprised between 20 and 100°C.

2. The process according to claim 1, **characterised in that** the lignocellulosic materials are selected from wood, recycled wood, annual plants, agricultural residues and recovered paper and cardboard, preferably wood.

3. The process according to claim 1 or 2, **characterised in that** said hydrolysate also comprises sugar degradation products, wood extractables and polymers derived from lignin and/or derived from sugar degradation products.

4. The process according to any one of the preceding claims, **characterised in that** the used amount oxidant is comprised between 1 and 100% by mass relative to the amount of lignin (precipitated and dissolved), furfural, hydroxymethylfurfural and of coloured compounds present in the hydrolysate.

5. The process according to any one of the preceding claims, **characterised in that** the oxidant is selected from hydrogen peroxide, ozone, peracids, free radicals and chlorinated oxidants.

6. The process according to any one of the preceding claims, **characterised in that** the oxidant is selected from ozone and hydrogen peroxide.

7. The process according to any one of the preceding claims **characterised in that** the oxidation step is performed at a pH comprised between 1 and 13, preferably between 3.5 and 11.

8. The process according to any one of the preceding claims, **characterised in that** it comprises the following steps:
- implementation of a hydrolysate of lignocellulosic materials;
- optionally, adjustment of the pH, to obtain a pH comprised between 3.5 and 12;
- bringing the hydrolysate into contact with at least one oxidant;
- heating of the reaction medium, at a temperature comprised between 20 and 100°C, preferably at a temperature lower than or equal to [in °C and in ascending order of preference]: 100; 90; 80; 70;
- recovery of a mixture of monosaccharides and/or oligosaccharides and/or polysaccharides containing less furfural and hydroxymethylfurfural and whose molecular weight of the lignin polymers and/or degradation products has been reduced;
- optionally, neutralisation of the residual oxidant.

9. The process according to any one of claims 1 to 7, **characterised in that** it comprises the following steps:
- heat treatment of lignocellulosic materials in the presence of water or water vapor to obtain a hydrolysate of lignocellulosic materials and a residue rich in cellulose, and optionally in lignin;
- separation of the hydrolysate of lignocellulosic materials from the residue rich in cellulose, for example by filtration;
- optionally, adjustment of the pH of the hydrolysate, to obtain a pH comprised between 3.5 and 12;
- bringing the hydrolysate into contact with at least one oxidant;
- heating the reaction medium, at a temperature comprised between 20 and 100°C, preferably at a temperature lower than or equal to [in °C and in ascending order of preference]: 100; 90; 80; 70;
- recovery of a mixture of monosaccharides and/or oligosaccharides and/or polysaccharides containing less furfural and hydroxymethylfurfural and whose molecular weight of the lignin polymers and/or degradation products has been reduced;
- optionally, neutralisation of the residual oxidant.

10. The process according to any one of the preceding claims, **characterised in that** the reaction is performed at the pH of the hydrolysate of lignocellulosic materials.

11. A mixture of monosaccharides and/or oligosaccharides and/or polysaccharides likely to be obtained by purification of a hydrolysate of lignocellulosic materials according to any one of claims 1 to 10, **characterised in that** it contains polymers derived from lignin, wood extractables and/or sugar degradation products, having a molecular mass by weight and/or by number reduced by at least 10%, preferably 25% and even more preferably 50%, relative to the polymers contained in the hydrolysate of lignocellulosic materials.

12. The mixture of monosaccharides and/or oligosaccharides and/or polysaccharides according to claim 11, **characterised in that** it comprises an amount of furfural and hydroxymethylfurfural reduced by at least 10%, preferably 25% and even more preferably 50%, relative to the amount of furfural and hydroxymethylfurfural present in the hydrolysate of lignocellulosic materials.
